# EUROPEAN PATENT APPLICATION

(11) **EP 4 717 186 A1**
(43) Date of publication of application: **01.04.2026**
(21) Application number: 24851758.3
(22) Date of filing: 02.08.2024
(51) Int. Cl.: A61B 8/12, A61B 1/00, A61B 1/045

(54) **PROCESSING DEVICE, PROCESSING METHOD, AND COMPUTER PROGRAM**

(30) Priority: 07.08.2023 JP 2023128754
(71) Applicant: Terumo Kabushiki Kaisha, Tokyo 151-0072 (JP)
(72) Inventor: ONIMURA, Yuji, Ashigarakami-gun, Kanagawa 259-0151 (JP)
(74) Representative: KIPA AB
(86) International application number: PCT/JP2024/027641
(87) International publication number: WO 2025/033333

(57) **Abstract**

A portable processing device, a processing method, and a computer program that facilitate grasping of remaining power are provided. The processing device includes a connection unit to which an image diagnosis catheter for a lumen organ having an ultrasound transmitter and receiver or an optical transmitter and receiver at a distal end is connected, a signal processing unit that processes a signal acquired from the ultrasound transmitter and receiver or the optical transmitter and receiver, a battery that supplies power to the ultrasound transmitter and receiver or the optical transmitter and receiver, a rotation drive unit of the image diagnosis catheter, and the signal processing unit, and a processing unit that calculates a number of procedures executable by a remaining charge of the battery according to a power use history for each procedure using the processing device and the remaining charge of the battery.

## Description

### Technical Field

The present invention relates to a portable processing device, a processing method, and a computer program.

### Background Art

In medical examination, an image obtained by directly imaging an image to be examined or imaging a measurement result using an electromagnetic wave is used for diagnosis. In particular, in the inspection of a lumen organ, various techniques using an image obtained by moving an image element into an organ are used.

Among lumen organs, in particular, image diagnosis of blood vessels is indispensable for safely and reliably performing operations such as percutaneous coronary intervention (PCI). For this reason, intravascular imaging techniques such as intravascular ultrasound (IVUS) using catheters, optical coherence tomography (OCT)/optical frequency domain imaging (OFDI), and the like are widely used in addition to angiography techniques for performing imaging from outside the body using contrast agents.

Patent Literature 1 discloses an ultrasonic probe in which the ultrasonic probe is of a wireless communication type and wireless charging is enabled in order to eliminate complexity of cable routing. Also in an imaging technique using a catheter such as OVUS or OCT, image transfer can be performed wirelessly and can be made portable.

### Citation List

### Patent Literature

Patent Literature 1: JP 2010-233826 A

### Summary of Invention

### Technical Problem

A portable electronic device has an indicator indicating remaining power of a built-in battery. The indicator often indicates remaining power as a percentage of full-charge capacity. However, what kind of processing can be completed with the remaining power is not clear to the user because the power consumption varies depending on the content of the processing.

An object of one aspect of the present disclosure is to provide a portable processing device, a processing method, and a computer program that make it easy to grasp remaining power.

### Solution to Problem

(1) A processing device according to an embodiment of the present disclosure includes a connection unit to which an image diagnosis catheter for a lumen organ having an ultrasound transmitter and receiver or an optical transmitter and receiver at a distal end is connected, a signal processing unit that processes a signal acquired from the ultrasound transmitter and receiver or the optical transmitter and receiver, a battery that supplies power to the ultrasound transmitter and receiver or the optical transmitter and receiver, a rotation drive unit of the image diagnosis catheter, and the signal processing unit, and a processing unit that calculates a number of procedures executable by a remaining charge of the battery according to a power use history for each procedure using the processing device and the remaining charge of the battery.
(2) In the processing device of (1), the processing unit may calculate a statistical value that is an average value, a median value, or a mode value of power consumption in one procedure using the processing device from the power use history, and calculate a number of procedures executable by the remaining charge of the battery on the basis of the remaining charge of the battery and the statistical value.
(3) In the processing device of (2), the processing unit may calculate the statistical value after executing processing of removing an abnormal value and/or an outlier from the power use history.
(4) In the processing device of (2) or (3), the processing unit may output a value obtained by dividing the remaining charge of the battery by the statistical value as a number of executable procedures.
(5) In the processing device according to any one of (1) to (4), the processing unit may calculate, as a numerical range, the number of procedures executable by the remaining charge of the battery on the basis of variation in power consumption for each time in the power use history.
(6) In the processing device according to any one of (1) to (5), the processing unit may correct the number of procedures executable by the remaining charge of the battery according to a deterioration state of the battery.
(7) In the processing device according to any one of (1) to (6), the processing unit may correct the number of procedures executable by the remaining charge of the battery according to an indoor temperature or an in-device temperature obtained from a temperature sensor.
(8) The processing device according to any one of (1) to (7) may further include a charging unit that charges the battery from an external power source, in which the processing unit may calculate a necessary charging time for the charging unit until a number of procedures executable by the remaining charge of the battery becomes equal to or more than a predetermined number of times on the basis of the remaining charge of the battery and the power use history.
(9) The processing device according to any one of (1) to (8) may further include a display unit, in which the processing unit may display on the display unit the calculated number of procedures executable by the remaining charge of the battery.
(10) The processing device according to any one of (1) to (9) may further include a wireless communication unit, in which the processing unit may output the calculated number of procedures executable by the remaining charge of the battery from the wireless communication unit to an external device.
(11) A processing method according to an embodiment of the present disclosure includes, by an image diagnosis apparatus, storing, as a history, a power consumption for each procedure using the image diagnosis apparatus, and calculating a number of procedures executable by a remaining charge of a battery according to a stored history of the power consumption and the remaining charge of the battery, the image diagnosis apparatus including a connection unit to which an image diagnosis catheter for a lumen organ having an ultrasound transmitter and receiver or an optical transmitter and receiver at a distal end is connected, and a signal processing unit that processes a signal acquired from the ultrasound transmitter and receiver or the optical transmitter and receiver, in which the image diagnosis apparatus receives power supply from the battery and activates the ultrasound transmitter and receiver or the optical transmitter and receiver, a rotation drive unit of the image diagnosis catheter, and the signal processing unit.
(12) A computer program according to an embodiment of the present disclosure causing a computer to execute processing includes storing, as a history, a power consumption for each procedure using the computer, and calculating a number of procedures executable by a remaining charge of a battery according to a stored history of the power consumption and the remaining charge of the battery, the computer including a connection unit to which an image diagnosis catheter for a lumen organ having an ultrasound transmitter and receiver or an optical transmitter and receiver at a distal end is connected, and a signal processing unit that processes a signal acquired from the ultrasound transmitter and receiver or the optical transmitter and receiver, in which the computer receives power supply from the battery and activates the ultrasound transmitter and receiver or the optical transmitter and receiver, a rotation drive unit of the image diagnosis catheter, and the signal processing unit.

### Advantageous Effects of Invention

According to the present disclosure, since the remaining power of a portable image diagnosis apparatus is output in units of the number of procedures using the image diagnosis apparatus itself, it is easy to be grasped, and it is possible to avoid a situation where the power is insufficient in the middle of a procedure.

### Brief Description of Drawings

Fig. 1 is a schematic view of an image diagnosis system including a processing device of a first embodiment.
Fig. 2 is a block diagram illustrating a configuration of the processing device of the first embodiment.
Fig. 3 is a block diagram illustrating a configuration of an information processing apparatus.
Fig. 4 is a flowchart illustrating an example of a processing procedure executed by the processing device.
Fig. 5 is a flowchart illustrating another example of a processing procedure executed by the processing device.
Fig. 6 is a diagram illustrating a display mode of remaining power in the processing device.
Fig. 7 is a bar graph illustrating a power use history.
Fig. 8 is a flowchart illustrating an example of a procedure of calculating the number of executable procedures by a processing device of a second embodiment.
Fig. 9 is a diagram illustrating a display mode of remaining power in the processing device of the second embodiment.
Fig. 10 is a block diagram illustrating a configuration of a processing device in a third embodiment.
Fig. 11 is a flowchart illustrating an example of a procedure of calculating the number of executable procedures by the processing device of the third embodiment.
Fig. 12 is a diagram illustrating a display mode of remaining power in the processing device of the third embodiment.
Fig. 13 is a flowchart illustrating another example of a processing procedure executed by a processing device of a fourth embodiment.
Fig. 14 is a diagram illustrating a display mode of remaining power in the processing device of the fourth embodiment.
Fig. 15 is a view illustrating an appearance of a processing device of a fifth embodiment.
Fig. 16 is a flowchart illustrating an example of a processing procedure of history display and history clear in the processing device of the fifth embodiment.
Fig. 17 is a schematic view of an image diagnosis system of a sixth embodiment.
Fig. 18 is a block diagram illustrating a configuration of a server apparatus.
Fig. 19A is a flowchart illustrating an example of a processing procedure executed by a processing device in a sixth embodiment.
Fig. 19B is a flowchart illustrating an example of a processing procedure executed by the processing device in the sixth embodiment.

### Description of Embodiments

Specific examples of an image diagnosis apparatus, an image diagnosis method, and a computer program according to an embodiment of the present invention will be described below with reference to the drawings.

### (First Embodiment)

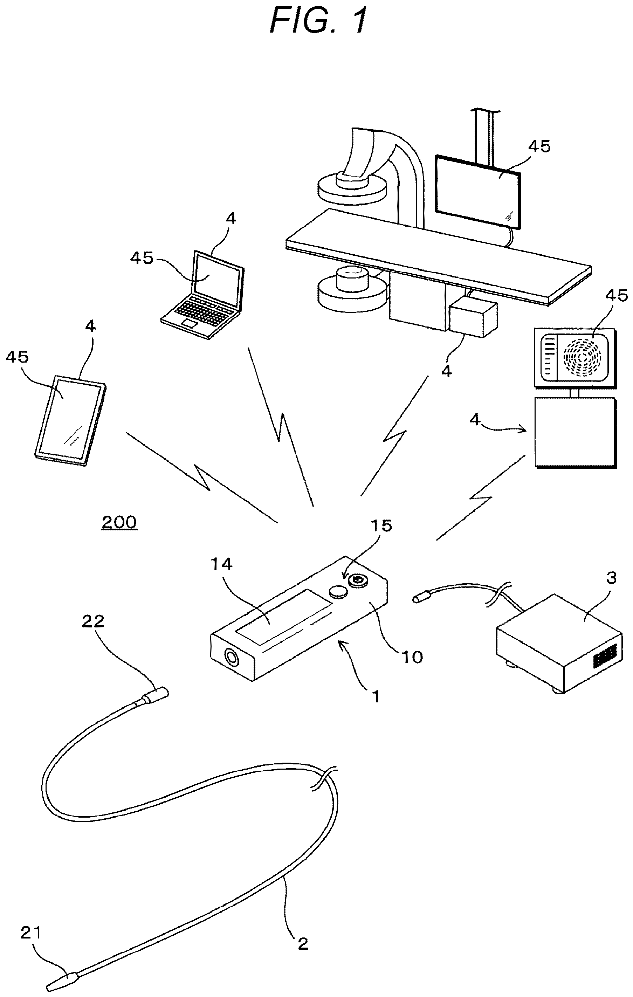

Fig. 1 is a schematic view of an image diagnosis system 200 including a processing device 1 of a first embodiment. The image diagnosis system 200 includes a processing device 1, a catheter 2, a light source device 3, and an information processing apparatus 4. When not in use, the processing device 1, the catheter 2, the light source device 3, and the information processing apparatus 4 are separated as illustrated in Fig. 1.

The catheter 2 is a medical flexible tube, and is used by being inserted into a lumen organ to be diagnosed. The catheter 2 is called an imaging catheter in which a shaft having a distal end to which an imaging device 21 is connected is inserted. The imaging device 21 and the shaft in the catheter 2 are driven to rotate in a circumferential direction by the processing device 1 connected via a connector 22 on a proximal end side.

The imaging device 21 of the catheter 2 is a dual type device including a transmitter and a receiver of waves of different wavelengths (ultrasonic wave or light), respectively. The imaging device 21 includes an IVUS circuit and an OCT circuit. The dual-type target is not limited to a combination of IVUS and OCT, and may be near-infrared spectroscopy or the like. The imaging device 21 is not limited to the dual type, and may be one using an imaging technique of only IVUS, only OCT, or only near-infrared spectroscopy.

A signal obtained by the imaging device 21 of the catheter 2 is output to the proximal end side of the catheter 2 via a signal line arranged in the shaft. The processing device 1 to which the catheter 2 is connected operates the imaging device 21 of the catheter 2, executes processing on a signal obtained from the imaging device 21, and transmits the signal to the information processing apparatus 4.

The light source device 3 is a device including an optical system for obtaining an OCT image using coherence of light. Although the light source device 3 is not essential, the light source device 3 will be described as being connected in order to employ a dual type of IVUS and OCT as the imaging device 21 in the first embodiment. The light source device 3 is disposed under a bed of a patient who uses the image diagnosis system 200. The light source device 3 receives power supplied from the power system and outputs light from the light source to the imaging device 21 of the catheter 2 via the processing device 1.

The information processing apparatus 4 is an apparatus that creates a tomographic image imaged from the inside of the lumen organ in which the catheter 2 is inserted from a signal obtained from the imaging device 21 via the processing device 1 and outputs information obtained by processing the created tomographic image. The information processing apparatus 4 is, for example, a medical apparatus such as an intravascular image diagnosis apparatus, an angiography apparatus, an external monitor, or an electrocardiograph. The information processing apparatus 4 outputs information obtained by the processing to the built-in display unit 45 or the externally connected display unit 45. The information processing apparatus 4 may be a smartphone, a tablet terminal, a laptop personal computer (PC), or a desktop PC, and functions as a medical apparatus such as an intravascular image diagnosis apparatus on the basis of a software program according to the application.

In the image diagnosis system 200 of the present disclosure, as illustrated in Fig. 1, the processing device 1 is not integrated with the display unit 45 or the information processing apparatus 4 installed at a place where a procedure is performed. The processing device 1 is configured to be separable so that the processing device 1 can be taken out from a predetermined place to an area where a procedure related to the catheter 2 is performed and used. When performing a procedure using the catheter 2, an operator such as a doctor or a medical technician connects the catheter 2 and the light source device 3 to the processing device 1, and uses the processing device 1 in a wireless communication connection with the information processing apparatus 4.

As illustrated in Fig. 1, the processing device 1 has an elongated substantially rectangular parallelepiped housing 10. The processing device 1 is preferably sized to be capable of being handled with one hand. The processing device 1 includes a built-in battery and is powered by the battery as a power source. Thus, the processing device 1 is detachable from the catheter 2 and the light source device 3, implements data input/output with the information processing apparatus 4 by wireless communication, and actualizes a portable image diagnosis apparatus related to the catheter 2.

In order to make the processing device 1 portable, it is necessary to take care not to interrupt the power supply from the built-in battery during the procedure while being easy to handle. Accordingly, in the processing device 1 of the first embodiment and each embodiment described below, the number of times that can be used by the remaining power of the battery built into the processing device 1 is displayed on the display unit 14 exposed from the housing 10 or the display unit 45 of the information processing apparatus 4 in units of procedures using the processing device 1. Here, the number of times in units of procedures represents how many times a state in which the power supply of the processing device 1 should be kept ON can be repeated regardless of the number of times of scan execution by the imaging device 21 in one procedure by inserting the catheter 2. In one procedure, scanning is often performed a plurality of times, for example, for observation of the state of the target blood vessel wall before indwelling of a stent or a balloon and for confirmation of the state of the blood vessel wall after indwelling of the stent or the balloon. The processing device 1 represents the remaining power in units of the number of times that can be used without interruption while the processing device 1 is in the power-on state from the insertion of the catheter 2 to the completion of the procedure.

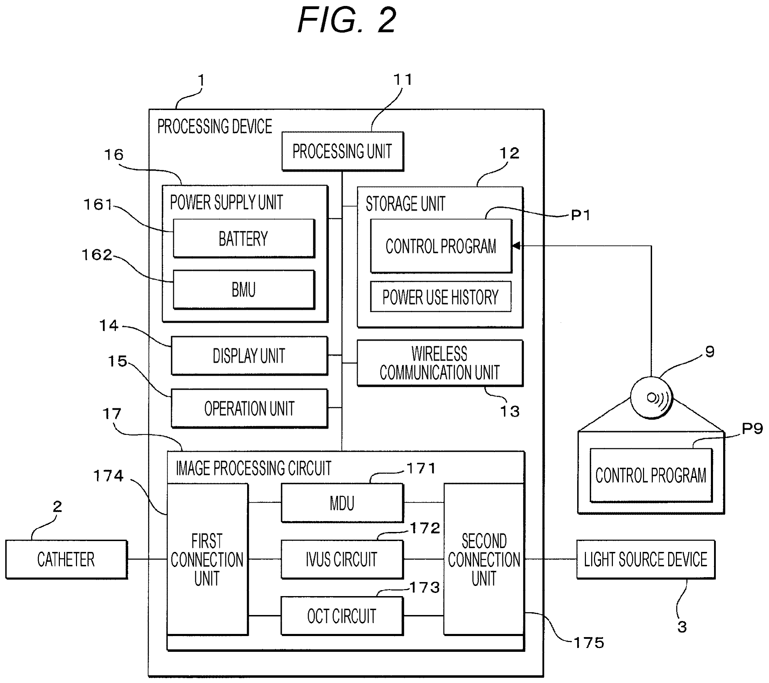

Hereinafter, processing for the configuration of the processing device 1 and the remaining charge of the battery will be described. Fig. 2 is a block diagram illustrating a configuration of the processing device 1 of the first embodiment. The processing device 1 includes a housing 10, a processing unit 11, a storage unit 12, a wireless communication unit 13, a display unit 14, an operation unit 15, a power supply unit 16, and an image processing circuit 17.

As illustrated in Fig. 1, the housing 10 has an elongated substantially rectangular parallelepiped shape. The housing 10 accommodates the processing unit 11, the storage unit 12, the wireless communication unit 13, the display unit 14, the operation unit 15, the power supply unit 16, and the image processing circuit 17 therein. The housing 10 exposes a part of each of the display unit 14, the operation unit 15, the power supply unit 16, and the image processing circuit 17.

The processing unit 11 includes a processor such as a central processing unit (CPU), a micro-processing unit (MPU), or a graphics processing unit (GPU). The processing unit 11 includes a memory such as a random access memory (RAM) or a read only memory (ROM), and the processor reads data from the memory and temporarily stores the processing result data in the memory to execute processing. The processing unit 11 controls each component on the basis of the control program P1 and the data stored in the storage unit 12, and executes processing of outputting the remaining power.

The storage unit 12 is a nonvolatile memory such as a flash memory or an SSD. The storage unit 12 stores data referred to by the processing unit 11, for example, a history of the amount of power used in one procedure as a power use history as described later. The storage unit 12 stores a control program P1.

The control program P1 stored in the storage unit 12 may be the control program P9 stored in the non-transitory storage medium 9 readable from the computer and read by the processing unit 11 and stored in the storage unit 12.

The wireless communication unit 13 is a communication module of near field communication, for example, Bluetooth (registered trademark). The processing unit 11 can transmit data to the information processing apparatus 4 by the wireless communication unit 13. The processing unit 11 may receive data from the information processing apparatus 4 by the wireless communication unit 13.

The display unit 14 is a display such as a liquid crystal display or an organic electro-luminescence (EL) display. The display unit 14 may be an LED lamp or a segment display. The processing unit 11 outputs information regarding the remaining power of a battery 161 to the display unit 14 based on the control program P1. The processing unit 11 may output information by a sound such as a beep sound using a sound output unit including a speaker together with the display unit 14.

The operation unit 15 is a physical UI capable of inputting and outputting to and from the processing unit 11. The operation unit 15 is, for example, a physical button. The operation unit 15 may be a touch panel built into the display unit 14. The operation unit 15 may use a sound input unit including a microphone. The operation unit 15 includes a power button and a scan button pressed to start processing by the image processing circuit 17. The operation unit 15 may further include a record button for recording signal data obtained by the image processing circuit 17. The operation unit 15 identifies which button has been selected and pressed, and notifies the processing unit 11 of the button.

The power supply unit 16 includes a battery 161 and a battery management unit (BMU) 162. The battery 161 is a rechargeable secondary battery. The battery 161 is, for example, a lithium ion battery. When activated by a power button included in the operation unit 15, the power supply unit 16 supplies power from the battery 161 to each component of the processing device 1. The battery 161 supplies power to a portion driven by the power of the catheter 2 connected at a first connection unit 174, for example, the imaging device 21. The battery 161 may be provided in a replaceable manner. The BMU 162 can output data of the output voltage, the current value, and the remaining power of the battery 161.

The power supply unit 16 can charge the battery 161 by receiving external power supply via a universal serial bus (USB) cable. The power supply unit 16 may include a power receiving coil and be capable of wireless charging.

The image processing circuit 17 executes operation control of the catheter 2 of the processing device 1 and processing on a signal obtained from the catheter 2. The image processing circuit 17 includes a motor drive unit (MDU) 171, an IVUS circuit 172, an OCT circuit 173, a first connection unit 174, and a second connection unit 175.

The MDU 171 is a drive device of the catheter 2. The MDU 171 is driven by a control signal from the processing unit 11. The MDU 171 controls the rotation of the catheter 2 and the operation of the imaging device 21 by driving an internal motor of the catheter 2 connected via the first connection unit 174 in accordance with the operation of the operator such as a doctor or a medical technician on the operation unit 15.

The IVUS circuit 172 drives an ultrasound transducer of the IVUS method of the imaging device 21, and acquires a signal obtained from the ultrasound probe for each radial scan. The IVUS circuit 172 outputs a signal for each scan with identification data for identifying the number of data, for example, every 360 degrees.

The OCT circuit 173 outputs light from the light source device 3 to drive a near-infrared light emitting unit of the imaging device 21, and acquires a signal obtained from a near-infrared sensor for each radial scan. The OCT circuit 173 outputs a signal for each scan with identification data for identifying the order of data, for example, every 360 degrees.

When the processing of the image processing circuit 17 is started, the processing unit 11 transmits a scanning signal for each of IVUS and OCT obtained from the IVUS circuit 172 and the OCT circuit 173 together with identification data from the wireless communication unit 13 to the information processing apparatus 4.

The connector 22 of the catheter 2 is detachably connected to the first connection unit 174. The first connection unit 174 includes a rotational drive mechanism for transmitting rotational drive by the MDU 171 to the shaft in the catheter 2. The second connection unit 175 is detachably connected to the light source device 3. The second connection unit 175 is connected to an optical fiber disposed in the image processing circuit 17. The second connection unit 175 outputs light from the connected light source device 3 to the OCT device in the catheter 2 via the optical fiber and the first connection unit 174.

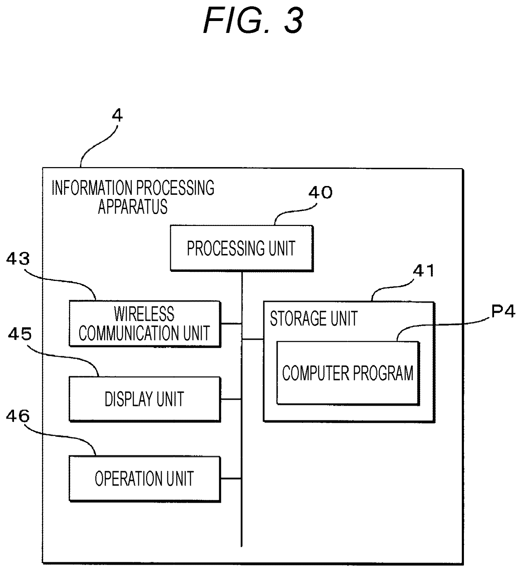

Fig. 3 is a block diagram illustrating a configuration of the information processing apparatus 4. The information processing apparatus 4 includes a processing unit 40, a storage unit 41, a wireless communication unit 43, a display unit 45, and an operation unit 46. The display unit 45 and/or the operation unit 46 may be externally attached and may input/output signals to/from the processing unit 40 via an input/output interface.

The processing unit 40 includes a processor such as one or more CPUs, MPUs, GPUs, GPGPU (general-purpose computing on graphics processing units), or Tensor Processing Units (TPUs). The processing unit 40 includes a non-transitory storage medium such as a random access memory (RAM), and executes computation based on a computer program P4 stored in the storage unit 41 while storing data generated during processing in the non-transitory storage medium.

The storage unit 41 is a non-volatile storage medium such as a hard disk or a flash memory. The storage unit 41 stores data read by the processing unit 40.

The wireless communication unit 43 is a communication device of near field communication. The wireless communication unit 43 only needs to be any communication device that implements wireless communication by a communication protocol corresponding to the wireless communication unit 13 of the processing device 1. The processing unit 40 receives data from the processing device 1 via the wireless communication unit 43.

As the display unit 45, a liquid crystal display panel, an organic electro luminescence (EL) display panel, or the like is used. The display unit 45 basically displays a medical image created by the processing of the processing unit 40 and information related to the medical image. The display unit 45 can output the information regarding the remaining power of the processing device 1 output by the processing unit 40 from the display unit 45 as described later.

The operation unit 46 is an input interface that receives an operation on the information processing apparatus 4. The operation unit 46 may be a keyboard, a mouse, or the like, or may be a touch panel, a soft key, a hard key, or the like built into the display unit 45. The operation unit 46 may receive an operation based on voice input. In this case, the operation unit 46 uses a microphone and a voice recognition engine.

In the image diagnosis system 200 configured as described above, the processing device 1 adds identification data to a signal for each scan obtained from the imaging device 21 for each of the IVUS and the OCT, and transmits the signal to the information processing apparatus 4. In the information processing apparatus 4, the processing unit 40 generates a rectangular image in which signals for each scan are aligned in the radial direction and arranged in a rectangular shape in the order based on the identification data, and a tomographic image obtained by performing polar coordinate transformation on the rectangular image, and displays the generated images on the display unit 45. The processing unit 40 executes image processing on the tomographic image and displays the processing result on the display unit 45 as well.

In the image diagnosis system 200, the processing unit 11 displays the remaining power on the display unit 14 or the display unit 45 in an easy-to-understand manner based on the control program P1 so that the procedure related to the catheter 2 can be executed without delay even in the portable processing device 1. Hereinafter, processing related to the display of the remaining power will be described with reference to a flowchart and a display example.

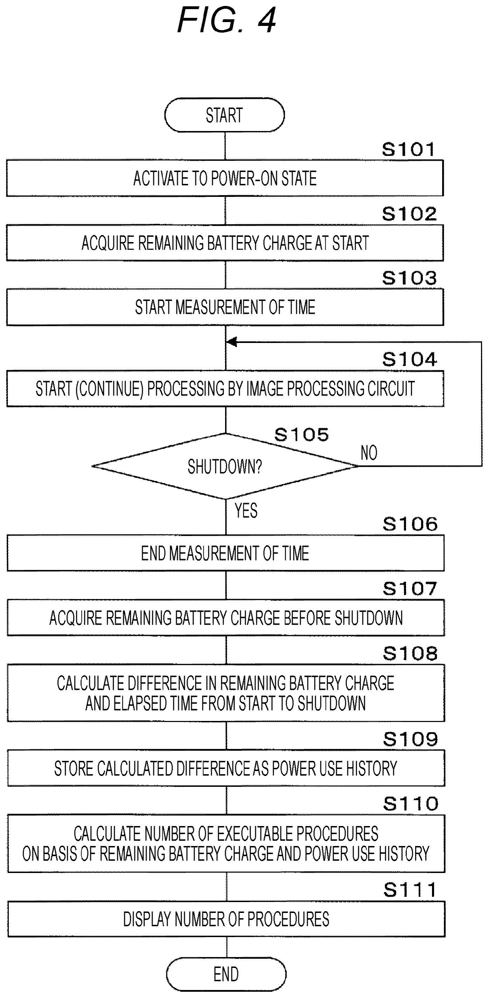

Fig. 4 is a flowchart illustrating an example of a processing procedure executed by the processing device 1. While being activated, the processing unit 11 executes the following processing according to the operation from the operator received by the operation unit 15.

When being activated to the power-on state by pressing the power button (S101), the processing unit 11 acquires the remaining battery charge at the time of activation from the BMU 162 of the power supply unit 16 (S102). The processing unit 11 starts measurement of time (S103), and starts or continues processing by the image processing circuit 17 by pressing a scan button (S104).

The processing unit 11 determines whether or not the scan is completed, the power button is pressed again, and shutdown is performed (S105). When it is determined that the power button has not been pressed (S105: NO), the processing unit 11 returns the process to S104 and continues the processing by the image processing circuit 17.

When the power button is pressed again and it is determined that shutdown is to be performed (S105: YES), the processing unit 11 ends the measurement of time (S106) and acquires the remaining battery charge before shutdown from the BMU 162 of the power supply unit 16 (S107).

The processing unit 11 calculates a difference in remaining battery charge from the time of activation to the shutdown and an elapsed time from the time of activation to the shutdown (S108), and the processing unit 11 stores the difference in remaining battery charge in the storage unit 12 as a power use history (S109). In S108, when acquiring the remaining battery charge from the BMU 162 as the charging rate, the processing unit 11 may calculate the remaining battery charge by multiplying the stored full charge capacity or may calculate the remaining battery charge as the rate. In S109, the processing unit 11 may overwrite the old history with the new history and store it at least a predetermined number of times.

The processing unit 11 calculates the number of executable procedures by the remaining battery charge based on the remaining battery charge acquired in S107 and the power use history stored in the storage unit 12 (S110).

In S110, for example, the processing unit 11 calculates a quotient obtained by dividing the remaining battery charge at the time of execution of S110 by the power consumption as the number of executable procedures, with the difference in the remaining battery charge from the time of activation to before the shutdown in the past use as the power consumption. In S110, the processing unit 11 may calculate an average value of the past power consumption (difference in remaining battery charge from the time of activation to before the shutdown), set the calculated average value as the power consumption, and use the average value as a divisor for dividing the remaining battery charge. The processing unit 11 may calculate not only the average value of the past power consumption but also a median value or a mode value and use the median value or the mode value as the power consumption.

After the power use history of a predetermined number of times or more remains in S110, the processing unit 11 may store the average value of the power consumption of the predetermined number of times in the storage unit 12 as the power consumption, and may use the average value as the power consumption without performing the calculation processing thereafter. Also in this case, the processing unit 11 may use not only the average value but also a statistical value such as a median value or a mode value. The processing unit 11 may store an average value, a median value, or a mode value obtained by removing an abnormal value and/or an outlier from the past power consumption in the power use history of the predetermined number of times or more in the storage unit 12 as power consumption, and refer to and use the power consumption.

In S110, the processing unit 11 may calculate a quotient obtained by dividing the usable time based on the remaining battery charge at the execution time of S110 by one usable time as the number of executable procedures, with the elapsed time from the activation to the shutdown in the past use as one use time. The usable time based on the remaining battery charge may be a specification value or may be calculated based on a past power use history. For example, the processing unit 11 can calculate the usable time corresponding to the remaining battery charge from the relationship between the power consumption of each time in the power use history and the elapsed time (use time) from the activation to before the shutdown.

The processing unit 11 displays the calculated number of procedures on the display unit 14 (S111) and terminates the processing, and the power is turned off. In S111, in a case where the calculated number of procedures is equal to or less than the predetermined number of times, the processing unit 11 may cause the display unit 14 to display a message indicating that the remaining number of times is small while outputting a beep sound from a speaker included in the processing device 1.

In the processing procedure illustrated in the flowchart of Fig. 4, the processing unit 11 calculates the amount of power used from when the power is turned on by pressing the power button until the power is turned off. However, it is not limited thereto, and the processing device 1 may calculate the amount of power used from pressing of the first scan button to before shutdown as power consumption for one time.

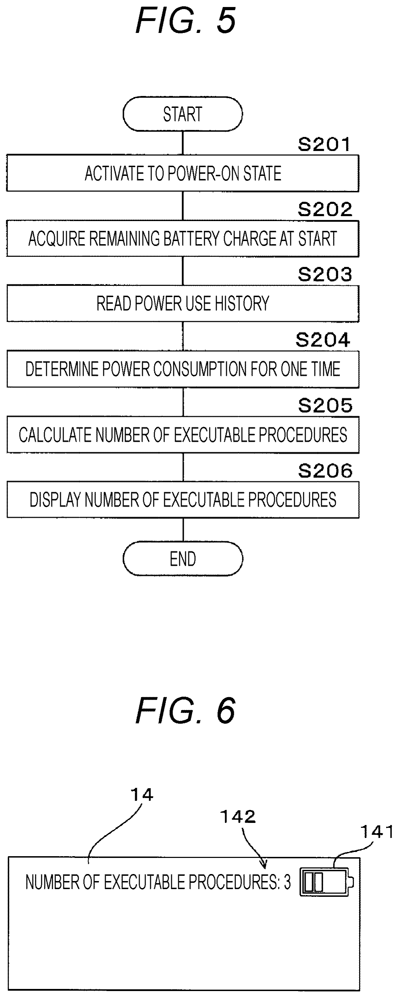

Fig. 5 is a flowchart illustrating another example of a processing procedure executed by the processing device 1. When being activated to the power-on state by pressing the power button (S201), the processing unit 11 acquires the remaining battery charge at the time of activation from the BMU 162 of the power supply unit 16 (S202).

The processing unit 11 reads the power use history stored in the storage unit 12 (S203), and determines power consumption for one time from the read power use history (S204). In S204, the processing unit 11 may determine, as the power consumption, a difference in remaining battery charge from the time of activation to before the shutdown in the most recent use, or may calculate an average value (median value or mode value) of the power consumption and determine the average value as power consumption for one time. In S204, after removing an abnormal value and/or an outlier from the past power consumption in the power use history, the processing unit 11 may calculate an average value (median value or mode value) and determine the average value as power consumption for one time. In S204, the processing unit 11 may determine, as power consumption for one time, a value already determined as the power consumption stored in the storage unit 12 by using the predetermined number of times or more.

The processing unit 11 calculates the number of executable procedures in units of the number of procedures that can be performed using the catheter 2 based on the remaining battery charge acquired in S202 and the power consumption determined in S204 (S205).

The processing unit 11 displays the calculated number of executable procedures on the display unit 14 (S206), and ends the display processing of the number of executable procedures. The processing unit 11 may enter the standby state and enter the OFF state by pressing the power button again, or the processing in the image processing circuit 17 may be started by pressing the scan button thereafter.

Fig. 6 is a diagram illustrating a display mode of the remaining power in the processing device 1. Fig. 6 illustrates display contents of the display of the display unit 14. The processing unit 11 displays the remaining charge in a manner as illustrated in Fig. 6 in S111 in the flowchart of Fig. 4 or S206 in the flowchart of Fig. 5. In the display example of Fig. 6, a gauge 141 indicating the ratio of the remaining battery charge and a numerical value 142 indicating the number of executable procedures "3" are displayed on the display unit 14. The remaining battery charge display by the gauge 141 is also performed in a conventional portable electronic device, but it is not clear whether or not the procedure using the processing device 1 can be performed once with the remaining charge. However, as illustrated in Fig. 6, the number of executable procedures is calculated and displayed, so that the operator can easily grasp the remaining power.

The gauge 141 illustrated in Fig. 6 is not essential, but by displaying not only the numerical value 142 of the number of executable procedures but also the gauge 141 as illustrated in Fig. 6, the operator can visually recognize the correspondence between the number of executable procedures and the remaining battery charge.

### (Second Embodiment)

In a second embodiment, the processing unit 11 of the processing device 1 calculates the number of executable procedures as a numerical range. The configuration of the image diagnosis system 200 of the second embodiment is similar to the configuration of the image diagnosis system 200 of the first embodiment except for details of processing described below. Therefore, among the configurations of the image diagnosis system 200 of the second embodiment, the same reference numerals are given to the configurations common to the first embodiment, and the detailed description thereof will be omitted.

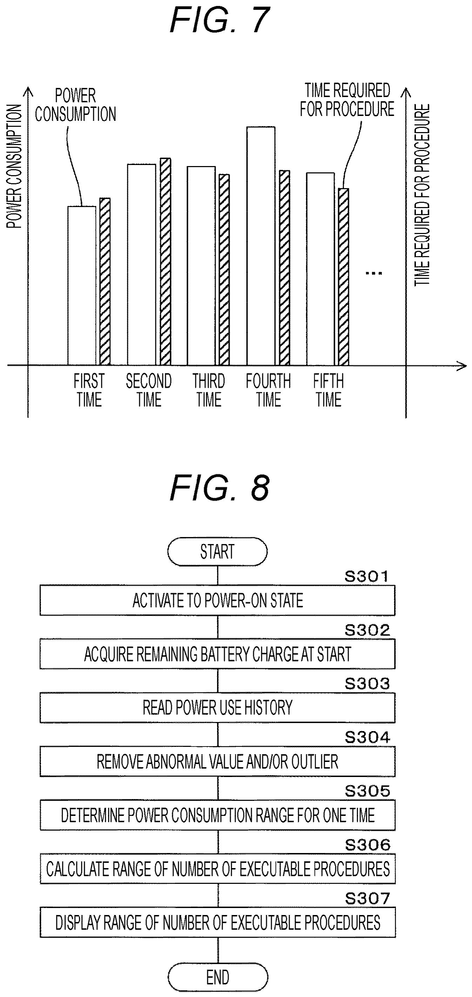

Fig. 7 is a bar graph illustrating a power use history. In Fig. 7, the horizontal axis represents the number of times, and the vertical axis represents the magnitude of the power consumption. Fig. 7 illustrates the amount of power used in each procedure (from the time of activation to shutdown) by the height of the bar graph for each instance. Along with the amount of power, the magnitude of the time required to complete one procedure is also illustrated on the right vertical axis for reference. As illustrated in Fig. 7, the power consumption at one time varies for each procedure. For example, when comparing the power consumption in the first procedure with the power consumption in the second and third procedures, the power consumption in the first procedure is about 20% less than the power consumption in the second and third procedures. The power consumption in the fourth procedure is about 20% larger than the power consumption in the second and third procedures, and the power consumption in the fifth procedure is about 10% larger. As described above, since there is variation, in the second embodiment, the processing unit 11 indicates the number of executable procedures using the processing device 1 as a numerical range not by a natural number in which a fractional part is rounded but by a fractional part in consideration of variation.

In the second embodiment, as illustrated in the flowchart of Fig. 4 of the first embodiment, the processing unit 11 of the processing device 1 stores the amount of power used from the time of activation to the shutdown as a power use history each time the power is used.

Fig. 8 is a flowchart illustrating an example of a procedure of calculating the number of executable procedures by the processing device 1 of the second embodiment. When being activated to the power-on state by pressing the power button (S301), the processing unit 11 acquires the remaining battery charge at the time of activation from the BMU 162 of the power supply unit 16 (S302).

The processing unit 11 reads the power use history stored in the storage unit 12 (S303), excludes an abnormal value and/or an outlier from the read power use history (S304), and determines a numerical range of power consumption for one time (S305). In S305, the processing unit 11 determines the numerical range of the power use history after removal of the abnormal value and/or the outlier as, for example, 320 to 440 [mAh].

By the processing of removing the abnormal value and/or the outlier in S304, it is possible to improve the accuracy by excluding the power consumption stored even when the power is turned on by test activation from the calculation target of the statistical value such as the average value.

The processing unit 11 calculates a numerical range of the number of procedures that can be performed in units of the number of executable procedures using the processing device 1 based on the remaining battery charge acquired in S302 and the numerical range of the power consumption determined in S305 (S306). In S306, for example, when the remaining battery charge is 1200 [mAh], which is 48% of the full-charge capacity 2500 [mAh] (specification value or estimated value), the processing unit 11 calculates the above-described numerical range of the number of executable procedures to be 2.7 to 3.8, where the numerical range of the power consumption for one time is 320 to 440 [mAh].

The processing unit 11 displays the calculated numerical range of the number of executable procedures on the display unit 14 (S307), and ends the display processing of the number of executable procedures. The processing unit 11 may enter the standby state and enter the OFF state by pressing the power button again, or the processing by the image processing circuit 17 may be started by pressing the scan button thereafter.

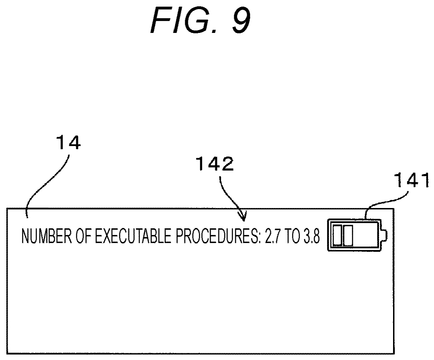

Fig. 9 is a diagram illustrating a display mode of the remaining power in the processing device 1 of the second embodiment. Similarly to Fig. 6 of the first embodiment, Fig. 9 illustrates display contents of the display of the display unit 14. When the processing illustrated in the flowchart of Fig. 4 is executed, the processing unit 11 displays the remaining charge in the processing of S111, and when the processing illustrated in the flowchart of Fig. 8 is executed, the processing unit 11 displays the remaining charge in the processing of S307 in a manner illustrated in Fig. 9. In Fig. 9, a numerical value 142 representing the number of executable procedures is illustrated as a numerical range including a decimal point. As illustrated in Fig. 9, in the second embodiment, a numerical value 142 of the number of executable procedures is indicated by a numerical range. As a result, the operator can more intuitively grasp the remaining power. In addition, by intentionally displaying variations, it can be expected to increase the credibility with respect to the number of executable procedures to be displayed.

### (Third Embodiment)

A processing device 1 in a third embodiment has a function of more accurately calculating the number of procedures executable by the remaining charge of the battery. The configuration of the image diagnosis system 200 of the third embodiment is similar to the configuration of the image diagnosis system 200 of the first embodiment except for the configuration and processing contents described below. Therefore, among the configurations of the image diagnosis system 200 of the third embodiment, the same reference numerals are given to the configurations common to the first embodiment, and the detailed description thereof will be omitted.

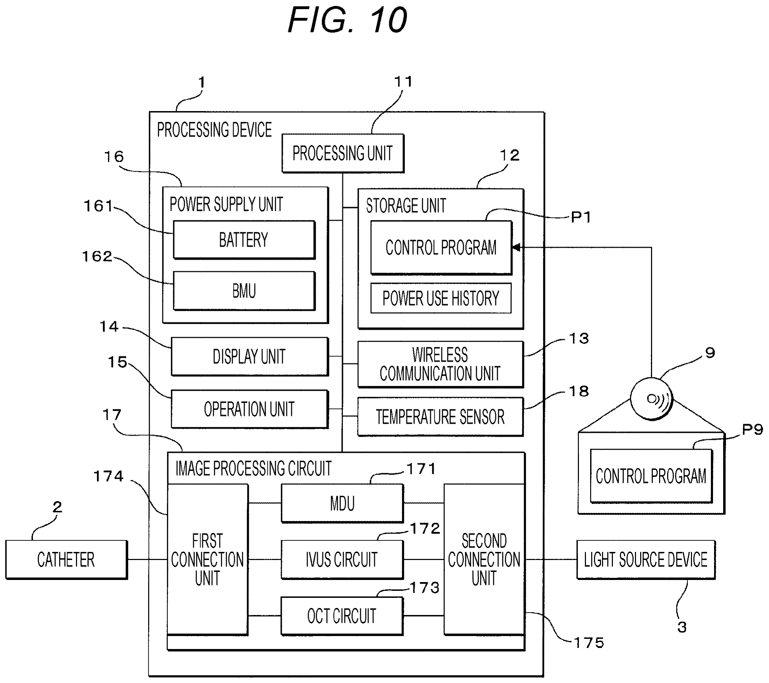

Fig. 10 is a block diagram illustrating a configuration of a processing device 1 in the third embodiment. In the third embodiment, the processing device 1 further includes a temperature sensor 18. The temperature sensor 18 is accommodated in the housing 10 and measures an in-device temperature. The temperature sensor 18 outputs the measured temperature to the processing unit 11. The processing unit 11 may store the measured temperature together when storing the power consumption in the storage unit 12 as the power use history (S109).

In the third embodiment, the processing unit 11 has a function of estimating a deterioration state of the battery 161. When the remaining power of the battery 161 becomes close to 0 and charging is continuously performed until full charge, the processing unit 11 estimates the full charge capacity from the accumulation of the current amount detected by the BMU 162, and stores the full-charge capacity in the storage unit 12 in association with time information obtained from the built-in timer or the wireless communication unit 13. The processing unit 11 can estimate the deterioration state from the transition of the full charge capacity stored in the storage unit 12. The deterioration state estimation function may be implemented by the BMU 162 instead of the processing unit 11. In this case, the processing unit 11 can acquire the deterioration state from the BMU 162.

The power consumption of the battery 161 changes according to the temperature and the deterioration state. Therefore, the processing unit 11 of the processing device 1 in the third embodiment corrects the calculated number of executable procedures on the basis of the temperature data obtained from the temperature sensor 18 and the deterioration state of the battery 161.

In the third embodiment, the processing unit 11 of the processing device 1 stores the amount of power used from the activation to before the shutdown as a power use history as illustrated in the flowchart of Fig. 4 of the first embodiment each time the power is used. When the power amount is stored, the power amount may be stored after corrected to the power consumption at a specified temperature (for example, 25°C).

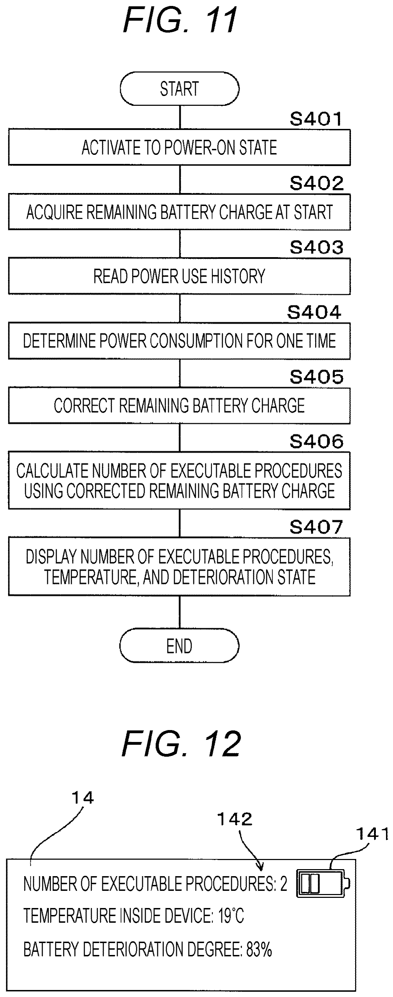

Fig. 11 is a flowchart illustrating an example of a procedure of calculating the number of executable procedures by the processing device 1 of the third embodiment. When being activated to the power-on state by pressing the power button (S401), the processing unit 11 acquires the remaining battery charge at the time of activation from the BMU 162 of the power supply unit 16 (S402).

The processing unit 11 reads the power use history stored in the storage unit 12 (S403), and determines power consumption for one time from the most recent predetermined number of use histories in the read power use history (S404). In S404, when the power consumption changes according to the deterioration of the battery 161, the processing unit 11 can calculate the number of executable procedures according to the deterioration by using the power consumption of the latest use history.

The processing unit 11 corrects the remaining battery charge acquired in S402 according to the acquired or estimated deterioration state and the temperature obtained from the temperature sensor 18 (S405). In S405, the processing unit 11 may perform correction according to a difference between the temperature data included in the power use history read in S403 and the temperature data obtained from the temperature sensor 18 at the time of S405. Instead of the process of S405, a process of equalizing the power consumption at the same temperature (for example, room temperature 25°C) may be executed in the process of S404.

The processing unit 11 calculates the number of procedures that can be performed in units of the number of executable procedures using the processing device 1 based on the remaining battery charge after the correction in S405 and the power consumption for one time determined in S404 (S406).

The processing unit 11 displays the calculated number of executable procedures, temperature, and deterioration state on the display unit 14 (S407), and ends the display processing of the number of executable procedures.

The processing unit 11 of the processing device 1 may perform correction according to the temperature and the deterioration state as illustrated in the flowchart of Fig. 11 also in the processing of S110 of the processing procedure illustrated in the flowchart of Fig. 4 of the first embodiment.

Fig. 12 is a diagram illustrating a display mode of the remaining power in the processing device 1 of the third embodiment. Fig. 12 illustrates display contents on the display of the display unit 14. When the processing illustrated in the flowchart of Fig. 4 is executed, the processing unit 11 displays the remaining charge in the processing of S111 in a manner illustrated in Fig. 12. When the processing illustrated in the flowchart of Fig. 11 is executed, the processing unit 11 displays the remaining charge in the processing of S407 in a manner illustrated in Fig. 12. As illustrated in Fig. 12, in the third embodiment, the measured temperature and the deterioration state of the battery 161 are illustrated together with a numerical value 142 indicating the number of executable procedures. The deterioration state is indicated by a numerical value indicating a ratio of the full charge capacity at that time point when the full charge capacity at the time of new is 100%.

In the third embodiment, the temperature sensor 18 is provided inside the processing device 1 to measure the in-device temperature, and the in-device temperature is used. The temperature used for the above-described correction is not limited thereto, and may be room temperature obtained from a separate temperature sensor provided at a predetermined place in the room via the wireless communication unit 13.

As described in the third embodiment, by determining the power consumption in consideration of the deterioration state and temperature that affect the power consumption of the battery 161 and calculating the number of practicable procedures, the operator can grasp the remaining power more accurately.

### (Fourth Embodiment)

A processing device 1 according to a fourth embodiment has a function of calculating a charging time of the battery 161 until the remaining battery charge reaches a level at which a procedure using the processing device 1 becomes possible. The configuration of the image diagnosis system 200 of the fourth embodiment is similar to the configuration of the image diagnosis system 200 of the first embodiment except for processing contents described below. Therefore, among the configurations of the image diagnosis system 200 of the fourth embodiment, the same reference numerals are given to the configurations common to the first embodiment, and the detailed description thereof will be omitted.

In the fourth embodiment, when detecting that the power supply unit 16 is connected to the power source in a wired manner or detecting that power reception is started by the power receiving coil, the processing unit 11 measures the time from the start of charging to the end of charging. The processing unit 11 calculates an increase rate of the charging rate per unit time or data corresponding thereto from a difference between the charging rate at the time of starting the charging and the charging rate at the time of ending the charging or the time of reaching the full charging and the elapsed time from the start of charging to the time of ending the charging (the time of reaching the full charging), and stores the increase rate or the data in the storage unit 12. The processing unit 11 may update the rate of increase or calculate and store a statistical value each time charging is performed.

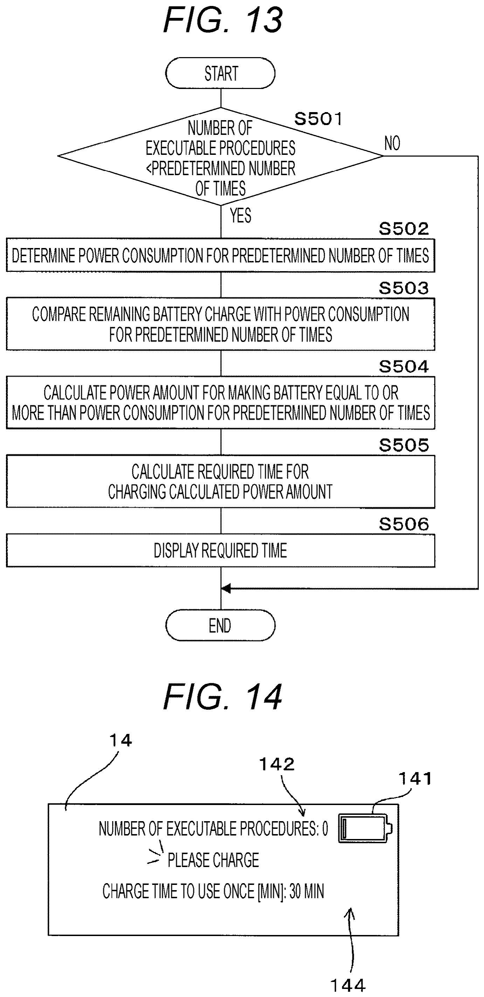

Fig. 13 is a flowchart illustrating another example of a processing procedure executed by the processing device 1 according to the fourth embodiment. Also in the fourth embodiment, as illustrated in Fig. 4 of the first embodiment, the processing unit 11 of the processing device 1 calculates the amount of power used each time it is used, and calculates the number of executable procedures in the next and subsequent times from the remaining battery charge. Then, the processing unit 11 executes the processing illustrated in Fig. 13 each time the number of executable procedures is calculated.

The processing unit 11 determines whether or not the calculated number of executable procedures is less than the predetermined number of times (S501). The predetermined number of times is, for example, once. When it is determined that the number of times is equal to or larger than the predetermined number of times (S501: NO), the processing unit 11 does not need the following processing and ends the processing. The processing unit 11 may execute the processing of S502 and subsequent steps even when the number of executable procedures is equal to or more than the predetermined number of times.

When it is determined that the calculated number of executable procedures is less than the predetermined number of times (S501: YES), the processing unit 11 determines the power consumption for the predetermined number of times (S502). The processing unit 11 compares the remaining battery charge with the power consumption for the predetermined number of times (S503), and calculates the power amount necessary to make the power of the battery 161 equal to or more than the power consumption for the predetermined number of times (S504).

The processing unit 11 calculates the required time for charging the calculated amount of power from the rate of increase in the charging rate per unit time stored in the storage unit 12 or data corresponding thereto (S505). The processing unit 11 displays the calculated required time on the display unit 14 (S506), and ends the processing. In S506, the processing unit 11 may display together the time required for charging for increasing the number of executable procedures by 1. In S506, the processing unit 11 may output a beep sound or a voice saying "Please charge." using a speaker as a warning sound for encouraging charging.

Fig. 14 is a diagram illustrating a display mode of the remaining power in the processing device 1 of the fourth embodiment. Fig. 14 illustrates display contents on the display of the display unit 14. The processing unit 11 displays the remaining charge in a mode as illustrated in Fig. 14 in the processing of S506 when the processing illustrated in the flowchart of Fig. 13 is executed. As illustrated in Fig. 14, in the fourth embodiment, a gauge 141 indicating the remaining power, a numerical value 142 indicating the number of executable procedures, and a numerical value 144 indicating the time required to set the number of executable procedures to the predetermined number of times (for example, 1 time) or more are illustrated. Furthermore, in the display example of Fig. 14, a text "Please charge" is displayed.

With the processing device 1 of the fourth embodiment, it is possible to grasp how long the processing device 1 should be charged in order to execute a procedure using the processing device 1 the predetermined number of times or more using the processing device 1, and the operator can perform preparation in advance in a planned manner. Although the time to full charge may be displayed, for example, for a procedure using the processing device 1 at least once, how many minutes to wait for the procedure to be usable is displayed in units of the number of times of the procedure, so that it can be grasped according to the use of the processing device 1 without depending on the intuition of the operator.

### (Fifth Embodiment)

In a fifth embodiment, a menu allowing viewing of the history of the power consumption of each processing device 1 is implemented. The configuration of the image diagnosis system 200 of the fifth embodiment is similar to the configuration of the image diagnosis system 200 of the first embodiment except for processing contents described below. Therefore, among the configurations of the image diagnosis system 200 of the fifth embodiment, the same reference numerals are given to the configurations common to the first embodiment, and the detailed description thereof will be omitted.

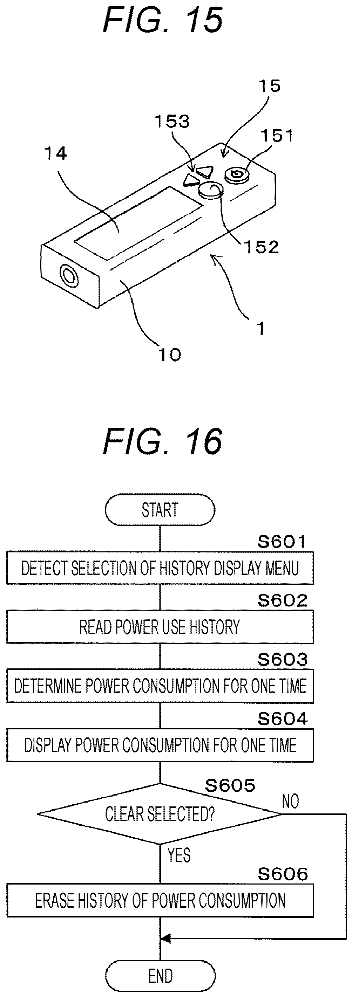

Fig. 15 is a view illustrating an appearance of a processing device 1 of the fifth embodiment. In the processing device 1 of the fifth embodiment, the operation unit 15 includes a scroll button 153 in addition to the power button 151 and the scan button 152. In the processing device 1 of the fifth embodiment, a menu can be called according to an operation on the operation unit 15. For example, when the power button 151 is pressed to turn on the power and then the scan button 152 is pressed for a long time, a menu is called up and displayed on the display unit 14, and the menu can be selected by the scroll button 153.

Fig. 16 is a flowchart illustrating an example of a processing procedure of history display and history clear in the processing device 1 of the fifth embodiment. When detecting that the menu for history display is selected (S601), the processing unit 11 of the processing device 1 reads the power use history of the storage unit 12 (S602).

The processing unit 11 determines power consumption for one time from the read power use history (S603). In S603, after removing an abnormal value and/or an outlier from the past power consumption in the power use history, the processing unit 11 may calculate an average value (median value or mode value) and determine the average value as power consumption for one time. The processing unit 11 displays the power consumption determined in S603 on the display unit 14 (S604).

The processing unit 11 determines whether or not the clearing of the power use history is selected from the menu (S605). When it is determined that clear is not selected (S605: NO), the processing unit 11 ends the process.

When it is determined that the clear is selected (S605: YES), the processing unit 11 erases the history of the power consumption stored in the storage unit 12 (S606), and ends the processing.

By enabling the processing procedure illustrated in Fig. 16 to be executed, the processing device 1 can be reset in a case where the calculation accuracy of the number of executable procedures is deteriorated from the history of the power consumption so far, such as a case where the type of the catheter 2 is changed or a case where the steps of the procedure are changed.

### (Sixth Embodiment)

In a sixth embodiment, the history of the power consumption is output to the external device. The configuration of the image diagnosis system 200 of the sixth embodiment is similar to the configuration of the image diagnosis system 200 of the first embodiment except for the configuration and processing contents described below. Therefore, among the configurations of the image diagnosis system 200 of the sixth embodiment, the same reference numerals are given to the configurations common to the first embodiment, and the detailed description thereof will be omitted.

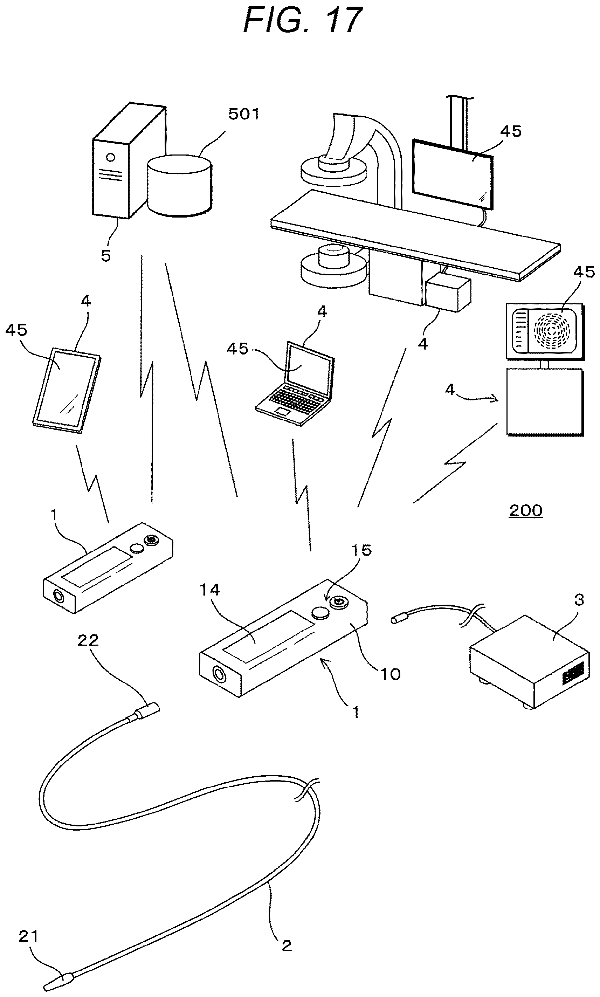

Fig. 17 is a schematic view of an image diagnosis system 200 of the sixth embodiment. The image diagnosis system 200 according to the sixth embodiment includes a plurality of processing devices 1 and a server apparatus 5. The server apparatus 5 is a server computer. The processing device 1 can transmit and receive data to and from the server apparatus 5 by communication in the same manner as the processing device 1 can transmit and receive data to and from the information processing apparatus 4 by wireless communication.

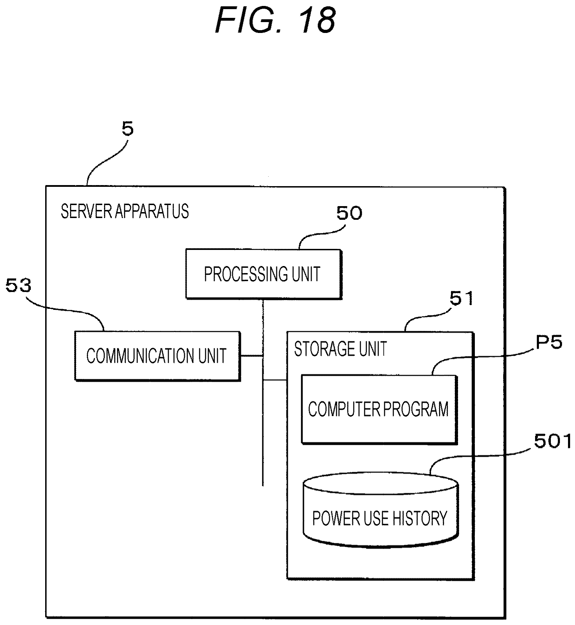

Fig. 18 is a block diagram illustrating a configuration of the server apparatus 5. The server apparatus 5 includes a processing unit 50, a storage unit 51, and a communication unit 53.

The processing unit 50 includes one or a plurality of processors such as a CPU, an MPU, a GPU, a GPGPU, or a TPU. The processing unit 50 includes a non-transitory storage medium such as a random access memory (RAM), and executes computation based on a computer program P5 stored in the storage unit 51 while storing data generated during processing in the non-transitory storage medium.

The storage unit 51 is a non-volatile storage medium such as a hard disk or a flash memory. The storage unit 51 stores data read by the processing unit 50. The storage unit 51 stores the power use history 501 transmitted from the processing device 1 for each processing device 1.

The communication unit 53 is a wired or wireless communication device. The communication unit 53 only needs to be, for example, a communication device that implements near field communication by a communication protocol corresponding to the wireless communication unit 13 of the processing device 1. The communication unit 53 is a wired device, and may be communicably connectable to the information processing apparatus 4 capable of wired communication. The processing unit 50 receives data from the processing device 1 via the communication unit 53.

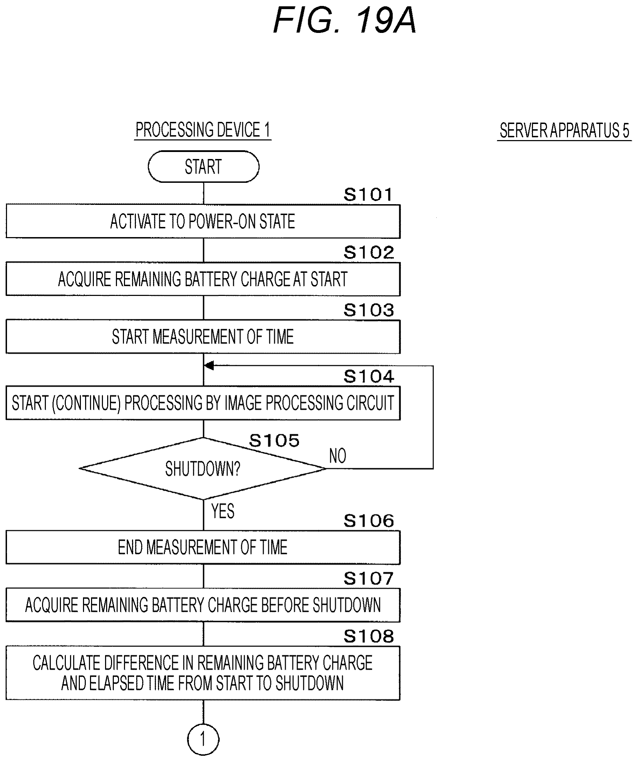

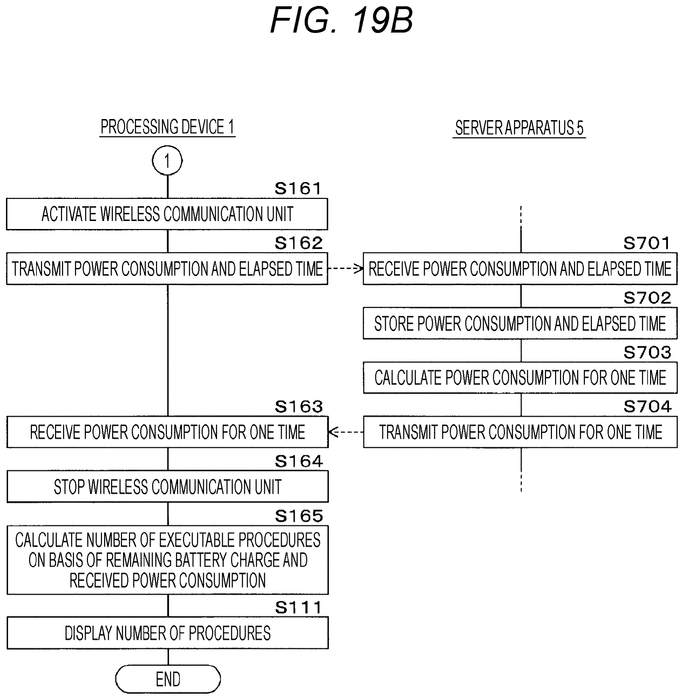

In the sixth embodiment configured as described above, the power consumption is stored in the server apparatus 5. Figs. 19A and 19B are flowcharts illustrating an example of processing procedures by the processing device 1 in the sixth embodiment. While being activated, the processing unit 11 of the processing device 1 executes the following processing according to the operation from the operator received by the operation unit 15. Among the processing procedures illustrated in Figs. 19A and 19B, the same step numbers are assigned to procedures common to the flowchart illustrated in Fig. 4 of the first embodiment, and detailed description thereof is omitted.

When the processing unit 11 calculates the difference in remaining battery charge from the time of activation to the shutdown and the elapsed time from the time of activation to the shutdown (S108), the wireless communication unit 13 is activated (S161). The processing unit 11 transmits the calculated power consumption and the elapsed time from the wireless communication unit 13 to the server apparatus 5 (S162). In S162, the processing unit 11 transmits the identification data of the processing device 1 together. In S162, the processing unit 11 may transmit the type of the catheter 2 used by the processing device 1, the type of the procedure, and the target case together.

In S162, the processing unit 11 may transmit the temperature obtained by the temperature sensor and the deterioration state of the battery 161.

In the server apparatus 5, when the power consumption for one time is received from the processing device 1 (S701), the processing unit 50 stores the power consumption as the power use history 501 in the storage unit 51 in association with the identification data of the processing device 1 (S702). When the type of the catheter 2, the type of the procedure, and the target case can be received from the processing device 1, the processing unit 50 stores these pieces of information in association with the power use history 501. The type of the catheter 2, the type of the procedure, and the target case may be acquired by the processing unit 50 from the information processing apparatus 4 functioning as an image diagnosis apparatus. The information processing apparatus 4 or the server apparatus 5 may store an execution schedule of image diagnosis for the lumen organ using the catheter 2, and may identify the type of procedure and the target case on the basis of the schedule.

The processing unit 50 calculates power consumption for one time from the power use history 501 of the storage unit 51 (S703) and transmits the power consumption to the processing device 1 (S704).

In S703, the processing unit 50 calculates, for example, a statistical value (average value, median value, or mode value) of the past power consumption for each of the plurality of processing devices 1 identified by the identification data. The processing unit 50 may calculate a statistical value of the past power consumption of the plurality of processing devices 1 used in the same facility.

In S703, the processing unit 50 may calculate the statistical value of the power consumption stored for the plurality of processing devices 1 for each type of the catheter 2, type of the procedure, and target case.

In S703, in a case where the in-device temperature of the processing device 1 and the deterioration state of the battery 161 are acquired, the processing unit 50 may correct the calculation result of the power consumption for one time using these pieces of information.

The processing unit 11 receives the power consumption for one time from the server apparatus 5 (S163), and stops the wireless communication unit 13 (S164). The processing unit 11 calculates the number of executable procedures in units of the number of procedures that can be performed using the processing device 1 based on the remaining battery charge acquired in S107 and the received power consumption for one time (S165).

The processing unit 11 displays the calculated number of executable procedures on the display unit 14 (S111), and ends the processing. Also in the sixth embodiment, in S111, in a case where the calculated number of executable procedures is equal to or less than the predetermined number of times, the processing unit 11 may cause the display unit 14 to display a message indicating that the remaining number of times is small while outputting a beep sound from a speaker included in the processing device 1.

In the image diagnosis system 200 of the sixth embodiment, by storing the power consumption in the server apparatus 5, the calculation resources used for calculating the power consumption for one time can be aggregated in the server apparatus 5. In addition, by statistically handling the power consumption in the plurality of processing devices 1, it can be expected to improve the calculation accuracy of the power consumption for each time according to the application of each facility.

The embodiments disclosed as above are illustrative in all respects and are not restrictive. The scope of the present invention is defined by the claims, and includes meanings equivalent to the claims and all modifications within the scope.

### Reference Signs List

- 1: Processing device
- 11: Processing unit
- 12: Storage unit
- 14: Display unit
- 16: Power supply unit
- 161: Battery
- 17: Image processing circuit
- 171: MDU
- 172: IVUS circuit
- 173: OCT circuit
- 174: First connection unit
- 175: Second connection unit
- P1: Control program (computer program)
- 2: Catheter (image diagnosis catheter)
- 4: Information processing apparatus
- 45: Display unit

## Claims

1. A processing device comprising: a connection unit to which an image diagnosis catheter for a lumen organ having an ultrasound transmitter and receiver or an optical transmitter and receiver at a distal end is connected; a signal processing unit that processes a signal acquired from the ultrasound transmitter and receiver or the optical transmitter and receiver; a battery that supplies power to the ultrasound transmitter and receiver or the optical transmitter and receiver, a rotation drive unit of the image diagnosis catheter, and the signal processing unit; and a processing unit that calculates a number of procedures executable by a remaining charge of the battery according to a power use history for each procedure using the processing device and the remaining charge of the battery.

2. The processing device according to claim 1, wherein
the processing unit calculates a statistical value that is an average value, a median value, or a mode value of power consumption in one procedure using the processing device from the power use history, and
calculates a number of procedures executable by the remaining charge of the battery on a basis of the remaining charge of the battery and the statistical value.

3. The processing device according to claim 2, wherein the processing unit calculates the statistical value after executing processing of removing an abnormal value and/or an outlier from the power use history.

4. The processing device according to claim 2 or 3, wherein the processing unit outputs a value obtained by dividing the remaining charge of the battery by the statistical value as a number of executable procedures.

5. The processing device according to any one of claims 1 to 4, wherein the processing unit calculates, as a numerical range, the number of procedures executable by the remaining charge of the battery on a basis of variation in power consumption for each time in the power use history.

6. The processing device according to any one of claims 1 to 5, wherein the processing unit corrects the number of procedures executable by the remaining charge of the battery according to a deterioration state of the battery.

7. The processing device according to any one of claims 1 to 6, wherein the processing unit corrects the number of procedures executable by the remaining charge of the battery according to an indoor temperature or an in-device temperature obtained from a temperature sensor.

8. The processing device according to any one of claims 1 to 7, further comprising a charging unit that charges the battery from an external power source, wherein
the processing unit calculates a necessary charging time for the charging unit until a number of procedures executable by the remaining charge of the battery becomes equal to or more than a predetermined number of times on a basis of the remaining charge of the battery and the power use history.

9. The processing device according to any one of claims 1 to 8, further comprising a display unit, wherein
the processing unit displays on the display unit the calculated number of procedures executable by the remaining charge of the battery.

10. The processing device according to any one of claims 1 to 9, further comprising a wireless communication unit, wherein
the processing unit outputs the calculated number of procedures executable by the remaining charge of the battery from the wireless communication unit to an external device.

11. A processing method comprising, by an image diagnosis apparatus:
storing, as a history, a power consumption for each procedure using the image diagnosis apparatus; and
calculating a number of procedures executable by a remaining charge of a battery according to a stored history of the power consumption and the remaining charge of the battery, the image diagnosis apparatus comprising: a connection unit to which an image diagnosis catheter for a lumen organ having an ultrasound transmitter and receiver or an optical transmitter and receiver at a distal end is connected; and a signal processing unit that processes a signal acquired from the ultrasound transmitter and receiver or the optical transmitter and receiver, wherein the image diagnosis apparatus receives power supply from the battery and activates the ultrasound transmitter and receiver or the optical transmitter and receiver, a rotation drive unit of the image diagnosis catheter, and the signal processing unit.

12. A computer program for causing a computer to execute processing including:
storing, as a history, a power consumption for each procedure using the computer; and
calculating a number of procedures executable by a remaining charge of a battery according to a stored history of the power consumption and the remaining charge of the battery, the computer comprising: a connection unit to which an image diagnosis catheter for a lumen organ having an ultrasound transmitter and receiver or an optical transmitter and receiver at a distal end is connected; and a signal processing unit that processes a signal acquired from the ultrasound transmitter and receiver or the optical transmitter and receiver, wherein the computer receives power supply from the battery and activates the ultrasound transmitter and receiver or the optical transmitter and receiver, a rotation drive unit of the image diagnosis catheter, and the signal processing unit.
